Europäisches Patentamt

European Patent Office

Office européen des brevets

(19)

(11) Numéro de publication : **0 517 573 A1**

# (12) DEMANDE DE BREVET EUROPEEN

(21) Numéro de dépôt : **92401493.9**

(22) Date de dépôt : **01.06.92**

(51) Int. Cl.⁵ : **A61K 31/70,** // (A61K31/70, 31:495)

(30) Priorité : 05.06.91 FR 9106783
02.12.91 FR 9114885

(43) Date de publication de la demande :
09.12.92 Bulletin 92/50

(84) Etats contractants désignés :
AT BE CH DE DK FR GB IT LI LU MC NL PT SE

(71) Demandeur : SYNTHELABO
22, Avenue Galilée
F-92350 Le Plessis Robinson (FR)

(71) Demandeur : INSTITUT NATIONAL DE LA
SANTE ET DE LA RECHERCHE MEDICALE
(INSERM)
101, rue de Tolbiac
F-75654 Paris Cédex 13 (FR)

(72) Inventeur : Puchelle, Edith, Inserm U 314
CHR. Maison Blanche, 45, rue Cognacq Jay
F-51092 Reims Cédex (FR)
Inventeur : Benali, Rachid, Inserm U 314
CHR. Maison Blanche, 45, rue Cognacq Jay
F-51092 Reims Cédex (FR)
Inventeur : Pierrot, Denis, Inserm U 314
CHR. Maison Blanche, 45, rue Cognacq Jay
F-51092 Reims Cédex (FR)
Inventeur : Girod, Sophie, Inserm U 314
CHR. Maison Blanche, 45, rue Cognacq Jay
F-51092 Reims Cédex (FR)
Inventeur : Zahm, Jean-Marie, Inserm U 314
CHR. Maison Blanche, 45, rue Cognacq Jay
F-51092 Reims Cédex (FR)
Inventeur : Moreau, Aline
133, rue Saint-Dominique
F-75007 Paris (FR)

(74) Mandataire : Thouret-Lemaitre, Elisabeth
SYNTHELABO Service Brevets 22, avenue
Galilée, B.P. Box 72
F-92352 LE PLESSIS ROBINSON CEDEX (FR)

(54) **Compositions pharmaceutiques pour le traitement des affections des voies respiratoires.**

(57)   Utilisation de l'adénosine-5′-triphosphate disodique pour la fabrication de moyens destinés à traiter les affections des voies respiratoires (ORL ou bronchiques) où il existe une deshydratation de l'épithélium et une hyperadhésivité du mucus.

EP 0 517 573 A1

Jouve, 18, rue Saint-Denis, 75001 PARIS

La présente invention a pour objet des compositions pharmaceutiques contenant de l'adénosine-5'-triphosphate disodique (ATP) ou de l'uridine-5'-triphosphate (UTP) utiles pour le traitement des affections des voies aériennes et de la mucoviscidose.

Plusieurs types cellulaires, notamment les cellules épithéliales, expriment des récepteurs pour l'ATP extracellulaire. L'activation de ces récepteurs s'accompagne de modifications du taux de calcium intracellulaire, médiateur et régulateur de multiples fonctions cellulaires épithéliales.

Ainsi, des concentrations micromolaires d'ATP sont capables d'entraîner une stimulation rapide de la fréquence des battements ciliaires des cellules épithéliales bronchiques (Girard P. Kennedy J. Calcium regulation of ciliary activity in rabbit tracheal epithelial explants and outgrowth. Eur. J. Cell Biol., 1986, 40 : 203-205). Cette action semble être liée à une augmentation du Ca++ intracellulaire (Villalon M., Hinds T.R. and Verdugo P. Intracellular Ca++ increases in respiratory ciliated cells following purinergic stimulation. J. Cell Biol., 1990, 2 : 170a).

Récemment, l'équipe du Professeur Boucher (Stutts M.J., Chinet T., Boucher R.C. Receptors for extracellular ATP are tighly coupled to outward rectifying Cl- channels in human airway epithelium. Presented to the N. Am. CF Conference Arlington - Oct. 1990) a rapporté que l'ATP extracellulaire est capable de provoquer l'ouverture des canaux chlore de l'épithélium bronchique et de stimuler la sécrétion intraluminale de chlore. Si comme l'évoque l'équipe de Boucher, l'ATP extracellulaire est capable de provoquer l'ouverture des canaux chlore, les cellules épithéliales en culture devraient être stimulables par cet ATP extracellulaire qui en augmentant, de façon active, la sécrétion intraluminale de chlore, augmenterait la sécrétion d'eau.

Pour étudier la sécrétion d'eau par les cellules épithéliales respiratoires, la Demanderesse a utilisé un système de culture en chambre bicompartimentale récemment développé par elle (Dupuit F., Jacquot J., Benali R., Hinnrasky J. and Puchelle E. Polarized secretion of proteins by human tracheal gland cells cultured on a permeable substrate. Am. Rev. Resp. Dis., 1991, 143 : A147).

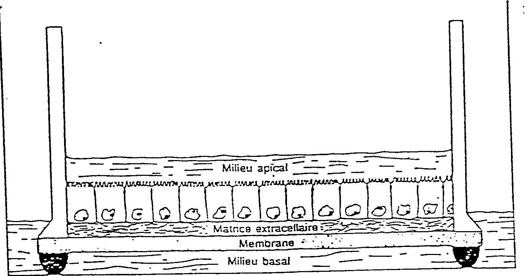

Les cellules épithéliales respiratoires de surface sont obtenues à partir de polypes nasaux humains par digestion enzymatique selon une technique développée par la Demanderesse (Chevillard M., Hinnrasky J., Pierrot D;, Zahm J.M., Klossek J.M., Puchelle E. Differenciation of human surface upper airway epithelial cells in primary culture on a floating collagen gel. Biol. Cell, 1990, 69 : 13a). Les cellules épithéliales respiratoires sont cultivées sur une membrane semi-perméable recouverte de collagène de types I et II en présence d'un milieu de culture sans sérum supplémenté en hormones et en facteurs de croissance. La culture des cellules sur cette membrane semi-perméable permet d'accéder séparément aux surfaces apicales et basolatérales des cellules (fig.1).

L'effet de l'ATP ou de l'UTP sur la sécrétion d'eau est étudié lorsque les cellules parviennent à confluence. L'état de confluence des cellules qui engendre une imperméabilité de la couche cellulaire est contrôlé par la mesure de la résistance cellulaire.

L'ATP ou l'UTP est apporté aux cellules par le compartiment apical dans un volume de 100 µl de solution de Ringer pH 7,4 à la concentration finale de $10^{-4}$M. Après deux heures d'incubation à 37°C, la variation du volume d'eau dans le compartement apical est évaluée par pesée. Il a été, dans cette étude, tenu compte des pertes de volume dues aux phénomènes de déshydratation dans les conditions expérimentales.

Par ailleurs, la Demanderesse a également étudié les effets de l'ATP et de l'UTP en présence d'amiloride.

L'étude est réalisée après incubation des cellules pendant 30 mn en présence ou non d'amiloride à la concentration de $10^{-4}$M.

Les résultats concernant les effets de l'ATP et de l'UTP, en présence ou non d'amiloride, sur la sécrétion d'eau par les cellules provenant de polypes de sujets sains sont très similaires et sont représentés dans les tableaux I et II ci-dessous :

## Tableau I

| | Témoin | Témoin + Amiloride | ATP* | ATP* + Amiloride |
|---|---|---|---|---|
| Δ% du volume initial | -1,26±0,90 (n=12) | +0,19±0,48 (n=7) | +1,65±0,74 (n=10) | +1,63±0,77 (n=7) |

Tableau II

|  | Témoin | Témoin + Amiloride | UTP* | UTP* + Amiloride |
|---|---|---|---|---|
| Δ% du volume initial | -1,47±0,51 (n=8) | +0,31±0,43 (n=8) | +1,68±0,52 (n=8) | +1,69±0,84 (n=8) |

Les valeurs représentent des moyennes ± écarts types ;

*p<0,001

n= nombre de cultures.

Les résultats obtenus ci-dessus montrent que l'amiloride seul bloque la réabsorption d'eau par les cellules épithéliales respiratoires de surface.

L'effet de l'ATP ou de l'UTP seuls (sans préincubation avec l'amiloride) sur les cellules épithéliales respiratoires se traduit par une augmentation significative (p < 0,001) du volume apical, donc par une sécrétion d'eau par les cellules.

La préincubation avec l'amiloride ne modifie pas de façon significative l'effet de l'ATP ou de l'UTP sur la sécrétion d'eau par les cellules épithéliales respiratoires.

Les résultats concernant l'effet de l'ATP et de l'UTP sur la sécrétion d'eau par les cellules provenant de polypes de sujets atteints de mucoviscidose (Cystic Fibrosis CF) sont représentés dans les tableaux III, IV et V suivants

Tableau III : CF ΔF 508 Homozygote

|  | Témoin | Témoin + Amiloride | ATP | ATP + Amiloride |
|---|---|---|---|---|
| Δ% du volume initial | -1,90±0,37 (n=4) | -0,08±0,40 (n=4) | +0,93±0,51 (n=4) | +0,80±0,60 (n=4) |

Tableau IV : CF mutation non identifiée

|  | Témoin | Témoin + Amiloride | ATP | ATP + Amiloride |
|---|---|---|---|---|
| Δ% du volume initial | -1,45+0,66 (n=6) | -0,64±0,55 (n=6) | +0,67±0,27 (n=6) | +0,82±0,57 (n=6) |

Tableau V : CF ΔF 508 Hétérozygote

|  | Témoin | Témoin + Amiloride | UTP | UTP + Amiloride |
|---|---|---|---|---|
| Δ% du volume initial | -1,70±1,05 (n=5) | -0,38±1,18 (n=5) | +0,42±0,24 (n=5) | +1,08±0,30 (n=5) |

Les résultats obtenus montrent que pour les cellules épithéliales respiratoires mucoviscidosiques (CF), l'amiloride ne semble pas bloquer totalement la réabsorption d'eau par ces cellules.

L'ATP ou l'UTP, seul ou en association avec l'amiloride, induisent une augmentation du volume apical et donc une sécrétion d'eau par les cellules.

L'ATP et l'UTP, en présence ou non d'amiloride, sont donc capables de provoquer l'ouverture des canaux chlore au niveau de l'épithélium respiratoire, de stimuler la sécrétion de chlore et d'eau par cet épithélium, et donc d'augmenter l'hydratation de l'épithélium respiratoire et du mucus des bronches ou des voies aériennes supérieures.

Chez les patients dont l'épithélium respiratoire est altéré par diverses pathologies (mucoviscidose, infections ou inflammations bronchiques ou ORL), les sécrétions sont fréquemment déshydratées. Or, au cours de ces pathologies, l'élimination des sécrétions repose essentiellement sur la clearance à la toux qui dépend de l'adhésivité du mucus.

La diminution de l'adhésivité du mucus est obtenue en particulier par son hydratation. L'ATP, l'UTP ou les associations ATP + amiloride ou UTP + amiloride qui peuvent augmenter l'hydratation du mucus, sont donc des agents de traitement potentiel de toutes les affections bronchiques ou ORL où il existe une deshydratation de l'épithélium et une hyperadhésivité du mucus, telles que (rhinites inflammatoires, infectieuses, allergiques, bronchites inflammatoires, infectieuses, allergiques ou toxiques, emphysème et mucoviscidose).

Les compositions pharmaceutiques de l'invention contenant de l'ATP, de l'UTP, l'association ATP + amiloride ou l'association UTP + amiloride, en association avec tout excipient approprié, peuvent donc être utilisées pour le traitement de ces affections.

Les compositions pharmaceutiques de l'invention peuvent être administrées par instillations ou sous forme de sprays pour les affections du domaine otorhinolaryngologique ; elles peuvent l'être sous forme de sprays ou par nébulisations pour les pathologies bronchiques.

**Revendications**

1. Utilisation de l'adénosine-5′-triphosphate disodique pour la fabrication de moyens destinés à traiter les affections des voies respiratoires (ORL ou bronchiques) où il existe une deshydratation de l'épithélium et une hyperadhésivité du mucus.

2. Utilisation de l'uridine-5′-triphosphate pour la fabrication de moyens destinés à traiter les affections des voies respiratoires (ORL ou bronchiques) où il existe une deshydratation de l'épithélium et une hyperadhésivité du mucus.

3. Utilisation de l'association adénosine-5′-triphosphate disodique et amiloride pour la fabrication de moyens destinés à traiter les affections des voies respiratoires (ORL ou bronchiques) où il existe une deshydratation de l'épithélium et une hyperadhésivité du mucus.

4. Utilisation de l'association uridine-5′-triphosphate et amiloride pour la fabrication de moyens destinés à traiter les affections des voies respiratoires (ORL ou bronchiques) où il existe une deshydratation de l'épithélium et une hyperadhésivité du mucus.

4

Milieu apical

Matrice extracellaire

Membrane

Milieu basal

EP 0 517 573 A1

Office européen
des brevets

## RAPPORT DE RECHERCHE EUROPEENNE

Numero de la demande

EP   92 40 1493

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X,P | FDC REPORTS: THE BLUE SHEET vol. 34, no. 35, 28 Août 1991, pages 4 - 5; 'aerosolized amiloride plus atp or utp therapy stimulates lung secretion in cystic fibrosis patients' | 2,4 | A61K31/70 //(A61K31/70, 31:495) |
| Y,P | * le document en entier *<br>--- | 1,3 | |
| Y | J. E. F. REYNOLDS 'Martindale, the extra pharmacopoeia' Février 1989 , THE PHARMACEUTICAL PRESS , LONDRES,GB * Page 1492, monographie 9203-v: "Adenosine Triphosphate"<br>--- | 1,3 | |
| X,P | THE NEW ENGLAND JOURNAL OF MEDICINE vol. 325, no. 8, 22 Août 1991, BOSTON pages 533 - 538; KNOWLES ET AL.: 'Activation by extracellular nucleotides of chlorine secretion in the airway epithelia of patients with cystic fibrosis' * page 537, colonne 2, ligne 44, alinéa 48 *<br>--- | 2 | |
| X,P | BRITISH JOURNAL OF PHARMACOLOGY vol. 103, no. 3, Juillet 1991, SALISBURY pages 1649 - 1656; MASON ET AL.: 'Regulation of transepithelial ion transport by extracellular atp in human normal and cystic fibrosis airway epithelium' *introduction*<br>----- | 1 | DOMAINES TECHNIQUES RECHERCHES (Int. Cl.5 )<br><br>A61K |

Le présent rapport a été établi pour toutes les revendications

| Lieu de la recherche | Date d'achèvement de la recherche | Examinateur |
|---|---|---|
| LA HAYE | 11 SEPTEMBRE 1992 | LEHERTE C.F.M. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

& : membre de la même famille, document correspondant

EPO FORM 1503 03.82 (P0402)

6